# EUROPÄISCHE PATENTSCHRIFT

(11) **EP 3 077 358 B1**
(45) Veröffentlichungstag und Bekanntmachung des Hinweises auf die Patenterteilung: **21.03.2018**
(21) Anmeldenummer: 14801931.8
(22) Anmeldetag: 14.11.2014
(51) Int. Cl.: C07C 45/50, C07C 45/62, C07C 45/74

(54) **VERFAHREN ZUR HERSTELLUNG VON 2-METHYLBUTANAL AUS DEN BEI DER HERSTELLUNG VON GEMISCHEN ISOMERER A, -UNGESÄTTIGTER DECENALE ANFALLENDEN NEBENSTRÖMEN**
METHOD FOR PRODUCING 2-METHYLBUTANAL FROM THE SECONDARY FLOWS ARISING IN THE PRODUCTION OF MIXTURES OF ISOMERIC , -UNSATURATED DECENALS
PROCÉDÉ DE PRÉPARATION DE 2-MÉTHYLBUTANAL À PARTIR DES FLUX SECONDAIRES PRODUITS LORS DE LA PRÉPARATION DE MÉLANGES DE DÉCÉNALS , -INSATURÉS ISOMÈRES

(30) Priorität: 05.12.2013 DE 102013020322
(43) Veröffentlichungstag der Anmeldung: 12.10.2016
(73) Patentinhaber: OXEA GmbH, 46147 Oberhausen (DE)
(72) Erfinder: THEUERKAUF, Jens, 47802 Krefeld (DE); STRUTZ, Heinz, 47445 Moers (DE)
(86) Internationale Anmeldenummer: PCT/EP2014/003057
(87) Internationale Veröffentlichungsnummer: WO 2015/082044

(56) Entgegenhaltungen:
- EP-A2- 0 217 159
- WO-A1-2005/028407
- WO-A1-2008/065171
- DE-A1- 2 155 672
- DE-A1-102009 027 978
- US-A- 4 426 542

## Beschreibung

Die vorliegende Erfindung betrifft ein Verfahren zur Herstellung von 2-Methylbutanal aus Nebenströmen, die bei der Herstellung von Gemischen isomerer α,β-ungesättigter Decenale anfallen. Pentanale, auch Valeraldehyde genannt, haben als Zwischenprodukte in der industriellen organischen Chemie wirtschaftliche Bedeutung erlangt. Sie kommen in vier verschiedenen Strukturisomeren als lineares n-Pentanal, verzweigtes 2-Methylbutanal, verzweigtes 3-Methylbutanal sowie als hochverzweigtes 2,2-Dimethylpropanal oder Pivalaldehyd vor. Pentanale können als solche verwendet werden, beispielsweise für die Herstellung von Riechstoffen oder in Form ihrer Derivatisierungsprodukte, wie Pentanole, Pentansäuren oder Pentylamine. Dabei können die Pentanale in reiner Isomerenform oder als Isomerengemisch verarbeitet werden (Weissermel, Arpe, Industrielle Organische Chemie, 3. Auflage, VCH Verlagsgesellschaft mbH, Weinheim, 1988, Seite 218; Schneidmeir, Chemiker-Zeitung, 96. Jahrgang (1972), Nr. 7, Seiten 383-387).

Aufgrund der reaktiven Aldehydgruppe können Pentanale im basischen Medium eine Aldoladditionsreaktion zu Pentanaldimerisierungsprodukten mit zehn Kohlenstoffatomen eingehen. Besitzt mindestens eines der Pentanalisomere zwei reaktive, zur Carbonylgruppe α-ständige Wasserstoffatome, kann das zunächst gebildete Aldoladditionsprodukt unter Wasserabspaltung in das α,β-ungesättigte Aldolkondensationsprodukt oder α,β-ungesättigte Decenal übergehen. Die Ausbildung der α,β-ständigen Doppelbindung relativ zum Carbonylkohlenstoffatom unter Wasserabspaltung wird häufig auch als Crotonisierung bezeichnet.

Bei der Aldolkondensation von Pentanalen können entweder strukturgleiche Pentanale in einer Selbstkondensation oder strukturverschiedene Pentanale in einer Co-Kondensation abreagieren. Unter den Pentanalen besitzt das lineare n-Pentanal die größte Reaktivität und bei der Aldolkondensation von n-pentanalhaltigen Pentanalgemischen wird vorzugsweise 2-Propylheptenal aus der Selbstkondensation von n-Pentanal gebildet. Die Aldoladditionsreaktion und Aldolkondensationsreaktion von Pentanalen wird üblicherweise in Gegenwart basischer Katalysatoren, wie wässrige Alkalihydroxidlösungen, durchgeführt.

Die Aldolkondensationsreaktion von Pentanalen zu Gemischen isomerer α,β-ungesättigter Decenale kann beispielsweise in einem Rohrreaktor, wie in DE 10 2009 001594 A1 und DE 199 57 522 A1 beschrieben, in einem Rührkessel, wie aus EP 0 366 089 A2 bekannt, oder in einer Reaktionsmischpumpe gemäß DE 10 2009 045 139 A1 durchgeführt werden. Je nach Anteilen an den isomeren Pentanalen in dem Einsatzgemisch werden Gemische mit strukturverschiedenen α,β-ungesättigten Decenale erhalten.

Auch US 4,426,542 A behandelt die Aldolisierung eines Pentanalgemisches in Gegenwart einer wässrigen Alkalihydroxidlösung und nachfolgende Hydrierung zu einem Gemisch isomerer Decanole. Nicht reagierte Aldehyde können vor der Hydrierstufe aus dem Decenalgemisch destillativ abgetrennt werden. Die isomeren Pentanale sind durch Hydroformylierungsreaktion linearer Butene zugänglich.

Die erhaltenen Gemische von isomeren α,β-ungesättigten Decenalen besitzen ebenfalls als organische Zwischenprodukte eine zunehmende wirtschaftliche Bedeutung. Die Kompletthydrierung der olefinischen Doppelbindung und der Aldehydgruppe liefert ein Gemisch strukturisomerer Decanole, die durch Veresterung mit aromatischen Di- oder Polycarbonsäuren sowie aliphatischen Dicarbonsäuren zu Weichmachern für thermoplastische Kunststoffe weiter verarbeitet werden. Die Abhängigkeit der Weichmachereigenschaften von der Zusammensetzung des Decanolgemisches wird beispielsweise in EP 0 366 089 A2 untersucht. Die Verwendung von isomeren Decanolgemischen als Weichmacheralkohole, die durch Kompletthydrierung von Gemischen isomerer α,β-ungesättigter Decenale erhalten werden, wird ebenfalls in DE 42 100 26 A1 und DE 43 33 324 A1 beschrieben.

α,β-ungesättigte Decenale können ebenfalls selektiv zu einem Gemisch isomerer Decanale hydriert werden, die aufgrund ihrer Aldehydreaktivität in weitere Folgeprodukte, insbesondere in Carbonsäuren durch Oxidation, umgewandelt werden können (DE 10 2009 027 978 A1). Isomere Decansäuren finden Anwendung zur Herstellung von Schmiermittelestern oder dienen zur Herstellung von Persäuren für Polymerisationsreaktionen.

Pentanale werden technisch durch Umsetzung von Butenen mit Synthesegas, einem Gemisch aus Kohlenmonoxid und Wasserstoff, in Gegenwart von Übergangsmetallverbindungen hergestellt. Man bezeichnet die Reaktion von Olefinen mit Synthesegas auch als Hydroformylierungsreaktion oder Oxo-Reaktion und man erhält bei der Hydroformylierung von Buten-1 neben dem geradkettigen n-Aldehyd n-Pentanal auch gewisse Anteile an dem iso-Aldehyd 2-Methylbutanal (Ullmann's Encyclopedia of Industrial Chemistry, 6th. Edition, 2003, Wiley-VCH Verlag GmbH & Co. KGaA, Weinheim, Vol. 2, Seiten 73-74; Vol. 25, Seiten 286-289).

Butene werden technisch durch die Dampfspaltung von Naphtha gewonnen. Da für die Herstellung von Gemischen isomerer α,β-ungesättigter Decenale die linearen Butene Buten-1 und Buten-2 von Bedeutung sind, wird üblicherweise aus dem Butenschnitt der Naphthaspaltung zunächst 1,3-Butadien unter Bildung von Raffinat I und anschließend Isobuten unter Bildung von Raffinat II abgetrennt (Weissermel, Arpe, Industrielle Organische Chemie, 3. Auflage, VCH Verlagsgesellschaft mbH, 1988, Seiten 71-79). Für die nachfolgende Hydroformylierungsreaktion setzt man überwiegend Raffinat II ein, in dem ein geringer Isobutenrestanteil zugelassen werden kann. In Sonderfällen kann auch Raffinat I mit einem hohen Isobutenanteil verarbeitet werden, gelegentlich auch ein an Buten-1 abgereichertes Raffinat II, das man auch als Raffinat III bezeichnet.

Die Hydroformylierungsreaktion kann sowohl in Anwesenheit als auch in Abwesenheit von komplexbildenden Verbindungen, beispielsweise in Gegenwart von Organophosphorverbindungen, durchgeführt werden. Nach EP 0 366 089 A2 arbeitet man in einer homogenen organischen Lösung unter Katalyse von Rhodium-Triphenylphosphin. Da im Allgemeinen ein möglichst hoher Anteil an n-Pentanal gegenüber 2-Methybutanal im gebildeten Pentanalgemisch angestrebt wird, wird die Hydroformylierungsreaktion häufig in Gegenwart von homogen gelösten Übergangsmetallkomplexen durchgeführt, die zunächst eine Isomerisierung des Butens-2 in Buten-1 ermöglichen, das dann im überwiegenden Maße zu n-Pentanal hydroformyliert wird. Für die isomerisierende Hydroformylierung eines Gemisches linearer Butene geeignete Rhodiumkomplexkatalysatoren werden beispielsweise in DE 102 25 282 A1 beschrieben, in denen die Komplexliganden ein Xanthengerüst besitzen.

Rhodiumkomplexkatalysatoren auf Basis von Bisphosphit-Liganden zusammen mit sterisch gehinderten sekundären Aminen, die ebenfalls für die isomerisierende Hydroformylierung eines Gemisches linearer Butene geeignet sind, werden in DE 10 2008 002 187 A1 behandelt. Auch zweistufige Verfahrensvarianten sind bekannt, beispielsweise nach DE 43 33 324 A1, DE 42 10 026 A1, DE 101 08 474 A1 und DE 101 08 475. In der ersten Stufe wird vorzugsweise Buten-1 umgesetzt während in der zweiten Stufe das Buten-2-haltige Abgas aus der ersten Stufe zu einem Gemisch aus n-Pentanal und 2-Methylbutanal hydroformyliert wird. Nach DE 43 33 324 A1 und DE 101 08 474 A1 kann die erste Hydroformylierungsstufe auch in Gegenwart wasserlöslicher Rhodiumkomplexkatalysatoren durchgeführt werden. Bei dieser Art der Reaktionsführung liegt eine flüssige, wässrige Katalysatorlösung neben der flüssigen, organischen Reaktionslösung vor, die nach Verlassen der Hydroformylierungszone auf einfache Weise voneinander durch Phasentrennung separiert werden können. Aufgrund des Vorliegens einer wässrigen und einer organischen flüssigen Phase bezeichnet man diese Art der Reaktionsführung auch als heterogenes Verfahren oder Zweiphasenverfahren.

Aufgrund des zunehmenden Bedarfs an Gemischen isomerer α,β-ungesättigter Decenale fallen bei der Hydroformylierungsreaktion des Einsatzbutengemisches erhebliche Mengen an 2-Methylbutanal und in gewissem Maße auch 3-Methylbutanal an, selbst wenn durch den Einsatz geeigneter Katalysatoren, die die isomerisierende Hydroformylierung unterstützen, die n-Pentanalbildung bevorzugt wird. Es besteht daher Bedarf an einem Verfahren, bei dem das bei der Herstellung von Gemischen isomerer α,β-ungesättigte Decenale anfallende 2-Methylbutanal in möglichst hoher Qualität und frei von Verunreinigungen gewonnen werden kann, so dass das anfallende 2-Methylbutanal möglichst vielseitig verwendet werden kann und so der Gesamtprozess zur Herstellung von Gemischen isomerer α,β-ungesättigter Decenale wirtschaftlicher gestaltet werden kann.

Die vorliegende Erfindung besteht daher in einem Verfahren zur Herstellung von 2-Methylbutanal aus den bei der Herstellung von Gemischen isomerer α,β-ungesättigter Decenale anfallenden Nebenströmen, dadurch gekennzeichnet, dass man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Pentanalgemisch in Gegenwart basischer Verbindungen zu einem Gemisch isomerer α,β-ungesättigter Decenale umsetzt; und
c) das gemäß Schritt b) erhaltene Gemisch in einen mit unumgesetztem 2-Methylbutanal angereicherten Stoffstrom und in einen mit einem Gemisch isomerer α,β-ungesättigter Decenale angereicherten Stoffstrom trennt; mit der Maßgabe,
   dass der gemäß Schritt c) abgetrennte und mit unumgesetztem 2-Methylbutanal angereicherte Stoffstrom mit Formaldehyd umsetzt wird.

Einsatzstoffe für das erfindungsgemäße Verfahren sind Kohlenwasserstoffgemische, die typischerweise keine oder sehr geringe Mengen an mehrfach ungesättigten Verbindungen und Acetylenverbindungen enthalten und mindestens eines der Olefine cis-Buten-2, trans-Buten-2 und Buten-1 enthalten. Darüber hinaus können in dem Einsatzgemisch wechselnde Anteile an Isobuten zugegen sein. Solche Einsatzgemische stehen als Raffinat I, Raffinat II oder Raffinat III technisch zur Verfügung.

Die Butenhydroformylierung kann dabei in homogener Variante in dem organischen Reaktionsmedium mit gelösten Übergangsmetallkatalysatoren der Gruppe VIII des Periodensystems der Elemente nach der unmodifizierten oder der mit Komplexliganden modifizierten Variante durchgeführt werden. In dem organischen Reaktionsmedium haben sich als besonders wirksame Lösungsmittel die höhersiedenden Kondensationsverbindungen der Pentanale, insbesondere die Trimeren, erwiesen, die als Nebenprodukte bei der Hydroformylierung anfallen, sowie ihre Mischungen mit den herzustellenden Pentanalen, so dass ein weiterer Lösungsmittelzusatz nicht unbedingt erforderlich ist. In einigen Fällen kann sich jedoch ein Lösungsmittelzusatz als zweckmäßig erweisen. Als Lösungsmittel werden organische Verbindungen eingesetzt, in denen Ausgangsmaterial, Reaktionsprodukt und Katalysator löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether wie Tetrahydrofuran, Ketone oder Texanol® der Firma Eastman. Wird bei der homogenen Variante in Gegenwart von Komplexliganden gearbeitet, eignen sich Triarylphosphine, wie Triphenylphosphin (EP 0 366 089 A2), Diphosphine, beispielsweise solche auf Basis des Xanthengerüstes (DE 102 25 282 A1), Phosphite wie beispielsweise in US 4,599,206 beschrieben, oder Diphosphite, beispielsweise wie in EP 0 213 639 A2 und DE 10 2008 002 187 A1 beschrieben. Auch Mischungen von Komplexliganden, beispielsweise aus Triarylphosphinen mit Phosphiten oder Diphosphiten, wie aus WO 2010/117391 A1 bekannt, können in der Hydroformylierungsreaktion eingesetzt werden.

Durch die Wahl der Zusammensetzung des Buteneinsatzgemisches und der Hydroformylierungsbedingungen kann das Verhältnis von n-Pentanal zu 2-Methylbutanal gesteuert werden.

Wird ein möglichst hoher Anteil an n-Pentanal gegenüber 2-Methylbutanal im Hydroformylierungsgemisch angestrebt, empfiehlt sich neben einem Buten-1 reichen Einsatzstrom die Verwendung von modifizierten Übergangsmetallkatalysatoren, die zunächst eine Isomerisierung des Restgehalts an Buten-2 in Buten-1 bewirken, das dann im überwiegenden Maße zu n-Pentanal hydroformyliert wird. In einer Ausgestaltung des erfindungsgemäßen Verfahrens können die aus DE 102 25 282 A1 bekannten Hydroformylierungskatalysatoren mit Komplexliganden auf Basis des Xanthengerüstes oder die aus EP 0 213 639 A2 oder DE 10 2008 002 187 A1 bekannten Hydroformylierungskatalysatoren auf Basis sterisch gehinderter Diphosphite eingesetzt werden.

Hohe Anteile an n-Pentanal können auch in einer zweistufigen Verfahrensvariante erhalten werden, die aus DE 101 08 474 A1 und DE 101 08 475 an sich bekannt ist. In der ersten Stufe, die ebenfalls nach der heterogenen Variante in Gegenwart von Wasser mit wasserlöslichen Komplexliganden, beispielsweise mit sulfonierten Phosphinen wie Triphenylphosphin mit unterschiedlichem Sulfonierungsgrad, durchgeführt werden kann, reagiert überwiegend Buten-1 in hoher Selektivität zu n-Pentanal und das mit Buten-2 angereicherte Abgas wird anschließend in einer zweiten Stufe unter isomersierenden Bedingungen zu einem Pentanalgemisch mit einem hohen n-Pentanalanteil umgesetzt. Durch Vereinigung der Stoffströme aus der ersten und zweiten Hydroformylierungsstufe kann ein Pentanalgemisch mit einem hohen Anteil n-Pentanal zu 2-Methylbutanal hergestellt werden.

Wird ein hoher Anteil an 2-Methylbutanal im Pentanalgemisch angestrebt, beispielsweise aufgrund von Marktgegebenheiten, oder er ergibt sich aufgrund der Zusammensetzung des Buteneinsatzgemisches, arbeitet man bevorzugt in Abwesenheit von Komplexliganden nach der unmodifizierten Fahrweise. Dabei bildet sich der aktive Hydroformylierungskatalysator aus dem Übergangsmetall oder der Übergangsmetallverbindung und Kohlenmonoxid. Es wird in der Fachliteratur angenommen, dass die Übergangsmetallverbindung HM(CO)₄ die katalytisch aktive Übergangsmetallspezies bei der unmodifizierten Übergangsmetallkatalyse ist.

Mit zunehmendem Isobutengehalt in dem Buteneinsatzgemisch nimmt auch der Anteil an 3-Methylbutanal in dem Hydroformylierungsprodukt zu.

Bei der modifizierten Variante beträgt das molare Verhältnis von Übergangsmetall zu Komplexligand im Allgemeinen 1:1 bis 1:1000, es kann aber auch noch höher liegen. Bevorzugt setzt man das Übergangsmetall und den Komplexliganden in einem molaren Verhältnis von 1:3 bis 1:500, vorzugsweise 1:50 bis 1:300 ein. Die modifizierte Hydroformylierungsreaktion des Butengemisches wird üblicherweise bei Temperaturen von 50 bis 160°C und Drücken von 0,2 bis 15 MPa durchgeführt. Die Übergangsmetallkonzentration beträgt im Allgemeinen 10 bis 700 ppm, vorzugsweise 25 bis 500 ppm, bezogen auf die Reaktionsmischung.

Wird nach der unmodifizierten Variante gearbeitet, setzt man das Übergangsmetall in geringeren Mengen ein, im Allgemeinen in einer Menge von 1 bis 100 ppm, vorzugsweise 2 bis 30 ppm, bezogen auf die eingesetzte Butenmenge. Man arbeitet zweckmäßigerweise bei höheren Drücken im Bereich von 5 bis 70 MPa, vorzugsweise von 5 bis 60 MPa und insbesondere von 10 bis 30 MPa. Geeignete Reaktionstemperaturen bewegen sich im Bereich von 50 bis 180°C, bevorzugt von 50 bis 150°C und insbesondere von 100 bis 150°C.

Die Zusammensetzung des Synthesegases kann in weiten Grenzen variiert werden. Im Allgemeinen setzt man Gemische ein, in denen das Molverhältnis von Kohlenmonoxid zu Wasserstoff 5:1 bis 1:5 beträgt. Üblicherweise ist dieses Verhältnis 1:1 oder weicht von diesem Wert zugunsten von Wasserstoff nur wenig ab. Das Gemisch enthaltend lineare Butene kann als solches oder in Lösung mit organischen Lösungsmitteln, wie Kohlenwasserstoffen, der Reaktionszone zugeführt werden.

Vorzugsweise verwendet man als Übergangsmetalle der Gruppe VIII des Periodensystems der Elemente Kobalt, Rhodium, Iridium, Nickel, Palladium, Platin, Eisen oder Ruthenium und insbesondere Rhodium und Kobalt. Der modifizierte oder nicht modifizierte Übergangsmetallkatalysator bildet sich unter den Bedingungen der Hydroformylierungsreaktion aus den eingesetzten Übergangsmetallverbindungen, wie deren Salzen, wie Chloriden, Nitraten, Sulfaten, Acetaten, Pentanoaten oder 2-Ethylhexanoaten, deren Chalkogeniden, wie Oxiden oder Sulfiden, deren Carbonylverbindungen, wie M₂(CO)₈, M₄(CO)₁₂, M₆(CO)₁₆, M₂(CO)₉, M₃(CO)₁₂, deren Organoübergangsmetallverbindungen, wie Carbonylacetylacetonaten oder Cyclooctadienylacetaten oder -chloriden. Dabei kann die Übergangsmetallverbindung als Feststoff oder zweckmäßigerweise in Lösung eingesetzt werden. Als Übergangsmetallverbindung, die als Katalysatorvorstufe verwendet wird, eignet sich insbesondere Rhodiumpentanoat, Rhodiumacetat, Rhodium-2-ethylhexanoat oder Kobaltpentanoat, Kobaltacetat oder Kobalt-2-ethylhexanoat oder Co₂(CO)₈, Co₄(CO)₁₂, Rh₂(CO)₈, Rh₄(CO)₁₂ oder Rh₆(CO)₁₆ oder Cyclopentadienylrhodiumverbindungen, Rhodiumacetylacetonat oder Rhodiumdicarbonylacetylacetonat. Bevorzugt werden Rhodiumoxid und insbesondere Rhodiumacetat, Rhodium-2-ethylhexanoat und Rhodiumpentanoat eingesetzt.

Es ist aber auch möglich, den Übergangsmetallkatalysator in einer Vorcarbonylierungsstufe zunächst zu präformieren und ihn anschließend der eigentlichen Hydroformylierungsstufe zuzuführen. Die Bedingungen der Präformierung entsprechen dabei im Allgemeinen den Hydroformylierungsbedingungen.

Die Hydroformylierungsstufe kann sowohl diskontinuierlich als auch kontinuierlich durchgeführt werden. Die Abtrennung des gebildeten Pentanalgemisches von dem Hydroformylierungskatalysator erfolgt nach konventionellen Verfahren, beispielsweise durch Destillation bei der homogenen Verfahrensführung oder durch einfache Phasentrennung von der wässrigen Katalysatorlösung bei der heterogenen oder zweiphasigen Verfahrensführung.

Der Übergangsmetallkatalysator wird gegebenenfalls nach Zusatz von frischer Übergangsmetallverbindung sowie gegebenenfalls von Frischligand, falls nach der modifizierten Fahrweise gearbeitet wird, und nach Entnahme eines Teils der im Verlauf der Reaktion gebildeten Aldehydkondensationsprodukte in die Reaktionszone zurückgeführt.

Die Forderung nach einer bestimmten Zusammensetzung des erhaltenen Pentanalgemisches bezüglich der Isomeren n-Pentanal, 2-Methylbutanal und 3-Methylbutanal richtet sich nach den Marktgegebenheiten und kann durch die Zusammensetzung des Einsatzbutengemisches sowie durch die Wahl der Hydroformylierungsbedingungen gesteuert werden. Häufig wird ein Pentanalgemisch angestrebt, das im Allgemeinen mindestens 85 mol-% n-Pentanal, weniger als 15 mol-% 2-Methylbutanal und in Abhängigkeit vom Isobutengehalt weniger als 5 mol-% 3-Methylbutanal, vorzugsweise weniger als 1 mol-% und insbesondere weniger als 0,2 mol-% 3-Methylbutanal enthält, jeweils bezogen auf die Summe an Pentanalen. Aber auch Pentanalgemische mit einem höheren Anteil an 2-Methylbutanal können vom Markt gefordert werden.
Das nach der Hydroformylierungsstufe und nach Katalysatorabtrennung erhaltene Pentanalgemisch, das man auch als rohes Hydroformylierungsprodukt ansehen kann, wird, gegebenenfalls nach destillativer Aufarbeitung, anschließend einer Aldolkondensation zu den α,β-ungesättigten Decenalen unter Einwirkung basischer Katalysatoren unterworfen. Als Katalysatoren finden Alkalicarbonate oder Alkalihydroxide, insbesondere Verbindungen des Natriums oder des Kaliums und Amine, vorzugsweise tertiäre Amine, wie Triethylamin, Tri-n-propylamin, Tri-n-butylamin, jeweils als wässrige Lösungen Anwendung. Die Konzentration des basischen Katalysators in der wässrigen Lösung beträgt üblicherweise 0,1 bis 10 Gew.-%, vorzugsweise 0,1 bis 3 Gew.-%. Man arbeitet bei Temperaturen von 20 bis 160°C, insbesondere 40 bis 130°C und bei Normaldruck oder bei bis zu etwa 1 MPa erhöhtem Druck. Als Reaktionsgefäße eignen sich Rührkessel oder eine Rührkesselkaskade, ein Strömungsrohr oder eine Mischpumpe. Durch den Einsatz statischer Mischer kann die wässrige Katalysatorphase mit der organischen Aldehydphase intensiv vermischt werden.

Die Reaktionszeit beträgt wenige Minuten bis zu mehreren Stunden und ist insbesondere abhängig vom Katalysatortyp und von der Reaktionstemperatur. N-Pentanal besitzt die höchste Reaktionsgeschwindigkeit und das α,β-ungesättigte Selbstkondensationsprodukt 2-Propylheptenal wird als Hauptprodukt gebildet. 2-Methylbutanal reagiert mit n-Pentanal zu dem Co-Kondensationsprodukt 2-Propyl-4-methylhexenal. Falls 3-Methylbutanal zusätzlich noch anwesend ist, entstehen das Selbstkondensationsprodukt 2-Isopropyl-5-methylhexenal sowie die Co-Kondensationsprodukte mit n-Pentanal 2-Propyl-5-methylhexenal und 2-Isopropylheptenal und das Co-Kondensationsprodukt mit 2-Methylbutanal 2-lsopropy-4-methylhexenal. Da 2-Methylbutanal im Vergleich zum n-Pentanal und 3-Methylbutanal weniger reaktiv ist, wird nach beendeter Aldolkondensationsreaktion das erhaltene Rohgemisch neben den gebildeten isomeren α,β-ungesättigten Decenalen auch noch Anteile an nicht reagiertem 2-Methylbutanal enthalten. Die Anteile an 2-Methylbutanal in dem Reaktionsgemisch können durch die Reaktionsbedingungen, beispielsweise durch Wahl der Reaktionstemperatur oder durch Verwendung von Lösungsmitteln, die Phasentransfereigenschaften besitzen, wie die Oligomeren des Ethylenglykols oder des Propylenglykols, in der Aldolkondensationsreaktion gesteuert werden.

Anschließend wird das Gemisch aus den isomeren α,β-ungesättigten Decenalen und nicht umgesetzten Ausgangsstoffen destillativ aufgearbeitet. Zunächst werden als Nebenstrom die flüchtigen Komponenten 2-Methylbutanal, gegebenenfalls 3-Methylbutanal und Restmengen an n-Pentanal abgezogen. Anschließend wird getrennt davon das Gemisch isomerer α,β-ungesättigter Decenale von höheren Aldolkondensationsprodukten und Hochsiedern abdestilliert. Die Destillation erfolgt in konventionellen Destillationskolonnen, wobei die anzuwendenden Temperatur- und Druckbedingungen durch Routineversuche ermittelt werden können. Anschließend kann der abgetrennte mit 2-Methylbutanal angereicherte Stoffstrom und der abgetrennte mit dem Gemisch der isomeren α,β-ungesättigten Decenale angereicherte Stoffstrom in Folgeprodukte überführt werden.

Beispielsweise erfolgt eine Selektivhydrierung der Doppelbindung in der α,β-Position relativ zum Carbonylkohlenstoffatom unter Beibehaltung der Aldehydgruppe, die zu einem Gemisch an gesättigten isomeren Decanalen führt. Die Selektivhydrierung wird in bekannter Weise an geträgerten oder ungeträgerten Katalysatoren, die als hydrieraktive Komponente Palladium, Platin, Rhodium und/oder Nickel enthalten, durchgeführt. Vorzugsweise arbeitet man mit Palladiumkatalysatoren bei Temperaturen von 120 bis 180°C, vorzugsweise 140 bis 160°C und bei einem Druck von 1,5 bis 5 MPa, vorzugsweise bei 2 bis 3 MPa. Der erhaltene Hydrieraustrag wird anschließend von Leicht- und Schwersiedern destillativ gereinigt.

In einer Ausgestaltung des erfindungsgemäßen Verfahrens kann bereits das rohe Aldolisierungsgemisch aus isomeren α,β-ungesättigten Decenalen und nicht umgesetzten Pentanalen der Selektivhydrierung bei den zuvor genannten Bedingungen unterzogen werden, wobei ein Gemisch aus gesättigten isomeren Decanalen und den nicht reagierten Pentanalen erhalten wird. Die anschließende destillative Aufarbeitung ergibt einen flüchtigen Nebenstrom, in dem 2-Methylbutanal angereichert ist, und getrennt davon einen mit einem Gemisch aus isomeren Decanalen angereicherten Stoffstrom.

Die vorliegende Erfindung betrifft daher ebenfalls ein Verfahren zur Herstellung von 2-Methylbutanal aus den bei der Herstellung von Gemischen isomerer Decanale anfallenden Nebenströmen, dadurch gekennzeichnet, dass man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Pentanalgemisch in Gegenwart basischer Verbindungen zu einem Gemisch isomerer α,β-ungesättigter Decenale umsetzt;
c) das gemäß Schritt b) erhaltene Gemisch in Gegenwart eines Hydrierkatalysators mit Wasserstoff bei erhöhter Temperatur und erhöhtem Druck in ein Gemisch isomerer Decanale selektiv hydriert; und
d) das gemäß Schritt c) erhaltene Gemisch in einen mit unumgesetztem 2-Methylbutanal angereicherten Stoffstrom und in einen mit einem Gemisch isomerer Decanale angereicherten Stoffstrom trennt; mit der Maßgabe,
   dass der gemäß Schritt d) abgetrennte und mit unumgesetztem 2-Methylbutanal angereicherte Stoffstrom mit Formaldehyd umgesetzt wird.

Die durch Auftrennung, zweckmäßigerweise durch konventionelle Destillation, des rohen Aldolisierungsproduktes oder die nach Selektivhydrierung und Auftrennung des rohen Hydriergutes erhaltenen mit unumgesetztem 2-Methylbutanal angereicherten Stoffströme können nach verschiedenen Varianten aufgearbeitet werden und in verschiedene Folgeprodukte überführt werden.

Die erhaltenen mit unumgesetztem 2-Methylbutanal angereicherten Stoffströme enthalten als Nebenprodukt unumgesetzte Restgehalte an n-Pentanal und gegebenenfalls 3-Methylbutanal, je nach Isobutengehalt im Einsatzbutengemisch. Die genaue Zusammensetzung des mit unumgesetztem 2-Methylbutanal angereicherten Stoffstromes hängt von der Zusammensetzung des Einsatzbutengemisches und den Aldolisierungsbedingungen ab.

Üblicherweise wird 2-Methylbutanal, das einen Siedepunkt von 92°C bei Normaldruck besitzt, durch eine weitere Destillation aus dem mit unumgesetzten 2-Methylbutanal angereicherten Produktstrom von Leichtsiedern und Hochsiedern befreit. Auch Anteile von n-Pentanal mit einem Siedepunkt von 103°C bei Normaldruck können dabei nach konventionellen Destillationsverfahren an einer weiteren Kolonne mit 10 bis 100 Böden als Produkt hoher Reinheit abgetrennt werden. Sind in dem mit 2-Methylbutanal angereicherten Stoffstrom jedoch Restmengen an 3-Methylbutanal enthalten, reicht die Siedepunktsdifferenz zum 3-Methylbutanal mit einem Siedepunkt von 92,5°C bei Normaldruck für eine ausreichende Trennung bei vertretbarem Destillationsaufwand nicht aus. In diesem Falle wird 2-Methylbutanal zusammen mit 3-Methylbutanal abgetrennt. Falls das jeweilige Anwendungsgebiet Restmengen an 3-Methylbutanal in dem 2-Methylbutanal zulässt, kann das 2-Methylbutanal in dieser Qualität in Folgeprodukte überführt werden.

Werden jedoch 2-Methylbutanal und daraus herzustellende Folgeprodukte in nahezu 3-methylbutanalfreier Qualität mit einem Restgehalt an 3-Methylbutanal von weniger als 0,2 Gew.-%, bezogen auf den organischen Anteil, nachgefragt, wird der mit 2-Methylbutanal angereicherte Stoffstrom, der gegebenenfalls 3-Methylbutanal enthält, entweder nach vorheriger destillativer n-Pentanalabtrennung oder mit Restgehalten an n-Pentanal gemäß der Erfindung mit Formaldehyd in Gegenwart eines Aldolisierungskatalysators umgesetzt. Die Behandlung von α-alkylsubstituierten Aldehyden mit Formaldehyd zur Entfernung von Restmengen an Aldehyden mit zwei Wasserstoffatomen am α-Kohlenstoffatom relativ zur Carbonylgruppe im Zuge der Aufarbeitung wird auch als Methylenierungsreaktion bezeichnet und ist aus dem Stand der Technik an sich bekannt und wird beispielsweise in DE 3842186 A1 und DE 3744212 A1 beschrieben. Das 2-Methyl-/3-Methylbutanal und gegebenenfalls n-Pentanal enthaltende Gemisch wird mit Formaldehyd behandelt in Gegenwart eines Aldolisierungskatalysators, typischerweise mit einem Gemisch aus einem sekundären

Amin, beispielsweise einem Amin der allgemeinen Formel R¹-NH-R², wobei R¹ und R² gleich oder verschieden sind und für Akylreste mit 1 bis 12, vorzugsweise 3 bis 5 Kohlenstoffatomen stehen, und einer Monocarbonsäure mit 1 bis 10 Kohlenstoffatomen oder einer Di- oder Polycarbonsäure mit 2 bis 10 Kohlenstoffatomen. Vorzugsweise verwendet man als Aldolisierungskatalysator ein Gemisch aus Di-n-Butylamin und n-Buttersäure. Aber auch andere Aldolisierungskatalysatoren sind nicht ausgeschlossen. Formaldehyd wird zweckmäßigerweise als wässrige Lösung in handelsüblicher Konzentration verwendet, beispielsweise in 30-50 Gew.-%iger Konzentration, wobei das molare Verhältnis von Formaldehyd zu der Summe an Aldehyden mit zwei Wasserstoffatomen am α-ständigen Kohlenstoffatom relativ zur Carbonylgruppe 1 bis 2 beträgt. Die Umsetzung wird üblicherweise bei Temperaturen von 0 bis 100°C bei Eigendruck oder leichtem Überdruck durchgeführt. Als Reaktoren eignen sich die in der chemischen Verfahrenstechnik üblichen Aggregate, wie Rührkessel, Rührkesselkaskaden, Mischpumpen oder Strömungsrohre, die statische Mischer zur Intensivierung der Durchmischung enthalten können. In einer besonderen Verfahrensvariante wird das Gemisch enthaltend 2-Methylbutanal, 3-Methylbutanal und n-Pentanal in Gegenwart von Formaldehyd und dem Aldolisierungskatalysator destilliert, wobei nahezu 3-methylbutanalfreies und gegebenenfalls n-pentanalfreies 2-Methylbutanal als Hauptfraktion abdestilliert. Neben der organischen Phase fällt Wasser durch den Wassereintrag und durch die Bildung von Isopropylacrolein und gegebenenfalls n-Propylacrolein an.

Bei der Methylenierung bildet sich zunächst aus Formaldehyd und dem sekundären Amin im sauren Medium ein Mannich-Salz, das selektiv mit 3-Methylbutanal und n-Pentanal über die Bildung des α-Methylolderivats in Isopropylacrolein und n-Propylacrolein dehydratisiert, während 2-Methylbutanal unverändert bleibt. Isopropylacrolein besitzt einen Siedepunkt von 108,5°C bei Normaldruck und n-Propylacrolein siedet bei 117°C unter Normaldruck und beide Methylenierungsprodukte können von dem 2-Methylbutanal destillativ abgetrennt werden. Der 3-Methylbutanalgehalt im abgetrennten 2-Methylbutanal liegt dabei im Allgemeinen unter 0,2 Gew.-%, bezogen auf den organischen Anteil. Gegebenenfalls schließt sich nochmals eine Feindestillation des 2-Methylbutanals an.

Das erhaltene 2-Methylbutanal, über die Methylenierungsreaktion mit anschließender Destillation aufgereinigt, kann als solches verwendet werden, beispielsweise zur Herstellung von Riechstoffen.

2-Methylbutanal kann ebenfalls nach an sich bekannten Verfahren (Ullmann's Encyclopedia of Industrial Chemistry, 6th. Edition, 2003, Wiley-VCH Verlag GmbH & Co. KGaA, Weinheim, Vol. 6, Seiten 497-502) zu 2-Methylbuttersäure oxidiert werden. Die Oxidation erfolgt vorzugsweise in der Flüssigphase beispielsweise in Rohrreaktoren, die mit einem Anströmboden versehen sind. Als Oxidationsmittel eignen sich Sauerstoff oder sauerstoffenthaltende Gase. Man arbeitet bei Temperaturen von 20 bis 100°C, vorzugsweise 20 bis 80°C und üblicherweise bei Normaldruck, wobei auch Verfahrenseinstellungen bis zu einem Druck (absolut) bis 0,8 MPa nicht ausgeschlossen sind.

Es empfiehlt sich, die Umsetzung in Anwesenheit von Alkali- oder Erdalkalimetallsalzen schwacher Säuren durchzuführen. Insbesondere bei der Oxidation von α-verzweigten Aldehyden, bei denen das dem Carbonylkohlenstoffatom benachbarte Kohlenstoffatom die Verzweigung trägt, schlägt der Stand der Technik die Anwesenheit von geringen Mengen an Alkalimetallcarboxylaten zur Selektivitätsverbesserung vor (DE 950 007, DE 100 10 771 C1) vor. Zweckmäßigerweise wird die Oxidation in Gegenwart von 1 bis 30 mmol, vorzugsweise von 1 bis 15 mmol und insbesondere von 1 bis 8 mmol, je Mol Aldehyd, berechnet als Alkali- oder Erdalkalimetall, durchgeführt. Vorzugsweise arbeitet man in Gegenwart von Kalium oder Natrium.

Auch kann eine Kombination von Alkali- oder Erdalkalimetallcarboxylaten mit Übergangsmetallverbindungen, wie in EP 1 854 778 A1 behandelt, verwendet werden.

Wird ein isobutenfreies Einsatzbutengemisch für den Hydroformylierungsschritt verwendet, ist der erhaltene mit unumgesetzten 2-Methylbutanal angereicherte Stoffstrom 3-methylbutanalfrei, er kann jedoch Restmengen an n-Pentanal enthalten, das über die Methylenierungsreaktion zu n-Propylacrolein dehydratisiert. Die Oxidation, wie zuvor beschrieben, liefert dann 2-Methylbuttersäure.

2-Methylbutanal kann ebenfalls über die reduktive Aminierung in 2-Methylbutylamine umgewandelt werden. Dabei wird das 2-Methylbutanal mit Ammoniak, einem primären oder sekundären Amin mit Wasserstoff in Gegenwart eines gängigen Aminierungskatalysators umgesetzt, wobei primäre, sekundäre und tertiäre 2-Methylbutylamine gebildet werden. Der Aminierungsgrad wird durch den Überschuss an Ammoniak oder an dem Zweitamin, wie n-Butylamin oder 2-Ethylhexylamin, bestimmt, wobei ein hoher Ammoniaküberschuss die Bildung des primären 2-Methylbutylamins begünstigt.

Die reduktive Aminierung wird in fachüblichen Reaktoren, beispielsweise in Rohrreaktoren an festangeordneten Aminierungskatalysatoren bei Temperaturen im Bereich von 100 bis 200°C, vorzugsweise von 110 bis 150°C, und bei Drücken im Bereich von 0,1 bis 40 MPa, vorzugsweise von 0,5 bis 30 MPa durchgeführt. Vorzugsweise erfolgt die Umsetzung an geträgerten oder trägerfreien Nickel- oder Kobaltkatalysatoren, die noch zusätzlich Promotoren, wie Oxide des Calciums, Bariums, Zinks, Aluminiums, Zirkoniums und Chroms enthalten können. Die Anwendung derartiger Aminierungskatalysatoren wird beispielsweise in DE 10 2012 014 395 B3 behandelt.

2-Methylbutanal kann ebenfalls zu 2-Methylbutanol hydriert werden. Die Hydrierung erfolgt in Gegenwart gängiger Hydrierkatalysatoren nach an sich bekannten Gasphasen- oder Flüssigphasenverfahren (Ullmann's Encyclopedia of Industrial Chemistry, 6th. Edition, 2003, Wiley-VCH Verlag GmbH & Co. KGaA, Weinheim, Vol. 2, Seite 24; Vol. 25, Seiten 286-289). Als Hydrierkatalysatoren eignen sich beispielsweise Nickel- oder Kupferkatalysatoren, bevorzugt Nickelkatalysatoren. Die Hydrierung erfolgt im Allgemeinen bei Wasserstoffdrücken von 1 bis 10 MPa und bei Temperaturen von 100 bis 180°C in der Gasphase, in der Flüssigphase oder in ihrer Kombination. In einer geeigneten Verfahrensführung wird in einer ersten Hydrierstufe am Kupferkatalysator in der Gasphase hydriert und nachfolgend in einer zweiten Hydrierstufe am Nickelkontakt in der Flüssigphase. Zur Reinigung wird das erhaltene 2-Methylbutanol destilliert. 2-Methylbutanol kann ebenfalls nach an sich bekannten katalytischen Verfahren durch Ammonolyse mit Ammoniak, primären oder sekundären Aminen in 2-Methylbutylamine überführt werden.

Die nach dem erfindungsgemäßen Verfahren erhaltenen Gemische isomerer Decanale können anschließend zu einem Gemisch isomerer Decansäuren oxidiert werden. Dieses Verfahren ist an sich bekannt und wird beispielsweise in DE 101 08 474 A1, DE 101 08 475 A1, DE 102 25 282 A1 und DE 10 2009 027 978 A1 behandelt. Die Oxidation des Decanalgemisches erfolgt entsprechend den zuvor genannten Bedingungen für die Oxidation von 2-Methylbutanal, vorzugsweise in Gegenwart von Alkali- oder Erdalkalimetallverbindungen. Aus dem nach der Oxidation anfallenden Rohsäuregemisch werden mittels Destillation unter üblichen Bedingungen Leicht- und Schwersieder entfernt. Der die Alkali- oder Erdalkalimetall-2-propylheptanoate und gegebenenfalls Übergangsmetalle enthaltende Destillationsrückstand wird abgetrennt und kann dem Einsatzaldehyd, gegebenenfalls nach Zugabe von frischen Alkali- oder Erdalkalimetall-2-propylheptanoaten oder Alkali- oder Erdalkalimetallverbindungen, die unter den Reaktionsbedingungen in die 2-Propylheptanoate übergehen, sowie gegebenenfalls von frischen Übergangsmetallverbindungen wieder zugeführt werden.

Das erhaltene Gemisch isomerer Decansäuren enthält je nach Zusammensetzung des Einsatzbutengemisches, den Reaktionsbedingungen und der gewählten Trennschärfe in den destillativen Aufarbeitungen wechselnde Anteile an 2-Propylheptansäure, 2-Propyl-4-methylhexansäure sowie an weiteren Decansäuren, die sich von den α,β-ungesättigten Decenalen ableiten lassen. Beispielsweise enthält ein Decansäuregemisch mehr als 90 mol-% 2-Propylheptansäure sowie weniger als 5 mol-% 2-Propyl-4-methylhexansäure, bezogen auf die Summe an Decansäuren und kann beispielsweise nach an sich bekannten Verfahren zur Herstellung von Derivaten wie den Vinylester, Carbonsäureestern, Säureanhydriden, Säurehalogeniden oder Säureamiden verwendet werden.

Das nach der Selektivhydrierung erhaltene Decanalgemisch kann ebenfalls über die reduktiven Aminierung in ein Gemisch isomerer Decylamine überführt werden, die analog zur reduktiven Aminierung von 2-Methylbutanal erfolgt. Das erhaltene Gemisch aus Decylaminen eignet sich besonders zur Verwendung als Korrosionsschutzmittel in Schmiermitteln, als Hilfsmittel in Gummiformulierungen, als Vulkanisationsbeschleuniger sowie als Additiv in Schmiermitteln, beispielsweise in Form ihrer Dithiocarbamate oder entsprechender Salze, wie Molybdän-, Zink- oder Natriumdithiocarbamaten.

Das über die Aldolisierungsreaktion erhaltene Gemisch isomerer α,β-ungesättigter Decenale kann auch komplett zu einem Gemisch isomerer Decanole hydriert werden. Ebenfalls kann das Decanalgemisch zu einem Gemisch isomerer Decanole hydriert werden. Die Hydrierung erfolgt analog den Bedingungen zur Hydrierung von 2-Methylbutanal und ist an sich bekannt und wird beispielsweise in EP 0 366 089 A2, DE 42 100 26 A1 und DE 43 33 324 A1 behandelt.

Zur Reinigung wird das erhaltene Gemisch isomerer Decanole destilliert. Es enthält je nach Zusammensetzung des Einsatzbutengemisches, den Reaktionsbedingungen und der gewählten Trennschärfe in der destillativen Aufarbeitung wechselnde Anteile an 2-Propylheptanol, 2-Propyl-4-methylhexanol sowie an weiteren Decanolen, die sich von den isomeren α,β-ungesättigten Decenalen ableiten lassen. Das erhaltene Gemisch isomerer Decanole kann als Alkoholkomponente für die Herstellung von Estern aromatischer und aliphatischer Di- und Polycarbonsäuren, wie Phthalsäure oder Phthalsäureanhydrid, Terephthalsäure, Trimellitsäure oder Adipinsäure verwendet werden. Diese Ester besitzen gegenüber thermoplastischen Kunststoffen, beispielsweise PVC, weichmachende Eigenschaften, die durch die Zusammensetzung des Decanolgemisches eingestellt werden können. Um besonders kälteflexible Kunststoffformkörper zu erhalten, sollte der 2-Propylheptanolanteil in dem zu veresternden Decanolgemisch möglichst hoch sein.

Das erhaltene Gemisch isomerer Decanole kann ebenfalls nach an sich bekannten katalytischen Verfahren durch Ammonolyse mit Ammoniak, primären oder sekundären Aminen in ein Gemisch isomerer Decylamine überführt werden.

Durch die erfindungsgemäße Herstellung von 2-Methylbutanal aus Nebenströmen, die bei der Herstellung von Gemischen isomerer α,β-ungesättigter Decenale anfallen, und den Folgeprodukten von 2-Methylbutanal und den Gemischen isomerer α,β-ungesättigter Decenale kann das Buteneinsatzgemisch besonders vorteilhaft wirtschaftlich verwertet werden.

## Patentansprüche

1. Verfahren zur Herstellung von 2-Methylbutanal aus den bei der Herstellung von Gemischen isomerer α,β-ungesättigter Decenale anfallenden Nebenströmen, **dadurch gekennzeichnet, dass** man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Pentanalgemisch in Gegenwart basischer Verbindungen zu einem Gemisch isomerer α,β-ungesättigter Decenale umsetzt; und
c) das gemäß Schritt b) erhaltene Gemisch in einen mit unumgesetztem 2-Methylbutanal angereicherten Stoffstrom und in einen mit einem Gemisch isomerer α,β-ungesättigter Decenale angereicherten Stoffstrom trennt; mit der Maßgabe,
dass der gemäß Schritt c) abgetrennte und mit unumgesetztem 2-Methylbutanal angereicherte Stoffstrom mit Formaldehyd umgesetzt wird.

2. Verfahren gemäß Anspruch 1, **dadurch gekennzeichnet, dass** die Umsetzung mit Formaldehyd in Gegenwart eines sekundären Amins und einer Mono-, Di- oder Polycarbonsäure erfolgt.

3. Verfahren gemäß Anspruch 2, **dadurch gekennzeichnet, dass** man bei der Umsetzung mit Formaldehyd destilliert.

4. Verfahren gemäß einem oder mehreren der Ansprüche 1 bis 3, **dadurch gekennzeichnet, dass** das gemäß Schritt c) erhaltene 2-Methylbutanal zu 2-Methylbuttersäure oxidiert, zu 2-Methylbutanol hydriert oder zu 2-Methylbutylaminen reduktiv aminiert wird.

5. Verfahren gemäß einem oder mehreren der Ansprüche 1 bis 3, **dadurch gekennzeichnet, dass** das gemäß Schritt c) erhaltene Gemisch isomerer α,β-ungesättigter Decenale zu einem Gemisch isomerer Decanole komplett hydriert oder zu einem Gemisch isomerer Decanale selektiv hydriert wird.

6. Verfahren gemäß Anspruch 5, **dadurch gekennzeichnet, dass** man das Gemisch isomerer Decanale zu einem Gemisch isomerer Decansäuren oxidiert oder zu einem Gemisch isomerer Decylamine reduktiv aminiert.

7. Verfahren zur Herstellung von 2-Methylbutanal aus den bei der Herstellung von Gemischen isomerer Decanale anfallenden Nebenströmen, **dadurch gekennzeichnet, dass** man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Pentanalgemisch in Gegenwart basischer Verbindungen zu einem Gemisch isomerer α,β-ungesättigter Decenale umsetzt;
c) das gemäß Schritt b) erhaltene Gemisch in Gegenwart eines Hydrierkatalysators mit Wasserstoff bei erhöhter Temperatur und erhöhtem Druck in ein Gemisch isomerer Decanale selektiv hydriert; und
d) das gemäß Schritt c) erhaltene Gemisch in einen mit unumgesetztem 2-Methylbutanal angereicherten Stoffstrom und in einen mit einem Gemisch isomerer Decanale angereicherten Stoffstrom trennt; mit der Maßgabe,
dass der gemäß Schritt d) abgetrennte und mit unumgesetztem 2-Methylbutanal angereicherte Stoffstrom mit Formaldehyd umgesetzt wird.

8. Verfahren gemäß Anspruch 7, **dadurch gekennzeichnet, dass** die Umsetzung mit Formaldehyd in Gegenwart eines sekundären Amins und einer Mono-, Di- oder Polycarbonsäure erfolgt.

9. Verfahren gemäß Anspruch 8, **dadurch gekennzeichnet, dass** man bei der Umsetzung mit Formaldehyd destilliert.

10. Verfahren gemäß einem oder mehreren der Ansprüche 7 bis 9, **dadurch gekennzeichnet, dass** man das gemäß Schritt d) erhaltene 2-Methylbutanal zu 2-Methylbuttersäure oxidiert, zu 2-Methylbutanol hydriert oder zu 2-Methylbutylaminen reduktiv aminiert.

11. Verfahren gemäß Anspruch 1 oder 7, **dadurch gekennzeichnet, dass** nach der Umsetzung mit Formaldehyd 2-Methylbutanal destillativ abgetrennt wird.

12. Verfahren gemäß Anspruch 11, **dadurch gekennzeichnet, dass** das erhaltene 2-Methylbutanal zu 2-Methylbuttersäure oxidiert, zu 2-Methylbutanal hydriert oder zu 2-Methylbutylaminen reduktiv aminiert wird.

13. Verfahren gemäß einem oder mehreren der Ansprüche 7 bis 9, **dadurch gekennzeichnet, dass** man das gemäß Schritt d) erhaltene Gemisch isomerer Decanale zu einem Gemisch isomerer Decanole hydriert, zu einem Gemisch isomerer Decansäuren oxidiert oder zu einem Gemisch isomerer Decylamine reduktiv aminiert.

## Claims

1. Process for preparing 2-methylbutanal from the secondary streams obtained in the preparation of mixtures of isomeric α,β-unsaturated decenals, **characterized in that**
a) a mixture comprising linear butenes is reacted in the presence of transition metal compounds of group VIII of the Periodic Table of the Elements with carbon monoxide and hydrogen at elevated temperature and elevated pressure to give a pentanal mixture;
b) the pentanal mixture obtained in step a) is converted in the presence of basic compounds to a mixture of isomeric α,β-unsaturated decenals; and
c) the mixture obtained in step b) is separated into a stream enriched with unconverted 2-methylbutanal and a stream enriched with a mixture of isomeric α,β-unsaturated decenals; with the proviso
that the stream which has been separated off in step c) and is enriched with unconverted 2-methylbutanal is reacted with formaldehyde.

2. Process according to Claim 1, **characterized in that** the reaction with formaldehyde is effected in the presence of a secondary amine and a mono-, di- or polycarboxylic acid.

3. Process according to Claim 2, **characterized in that** distillation is effected in the course of the reaction with formaldehyde.

4. Process according to one or more of Claims 1 to 3, **characterized in that** the 2-methylbutanal obtained in step c) is oxidized to 2-methylbutyric acid, hydrogenated to 2-methylbutanol or reductively aminated to 2-methylbutylamines.

5. Process according to one or more of Claims 1 to 3, **characterized in that** the mixture of isomeric α,β-unsaturated decenals obtained in step c) is completely hydrogenated to give a mixture of isomeric decanols or selectively hydrogenated to give a mixture of isomeric decanals.

6. Process according to Claim 5, **characterized in that** the mixture of isomeric decanals is oxidized to give a mixture of isomeric decanoic acids or reductively aminated to give a mixture of isomeric decylamines.

7. Process for preparing 2-methylbutanal from the secondary streams obtained in the preparation of mixtures of isomeric decanals, **characterized in that**
a) a mixture comprising linear butenes is reacted in the presence of transition metal compounds of group VIII of the Periodic Table of the Elements with carbon monoxide and hydrogen at elevated temperature and elevated pressure to give a pentanal mixture;
b) the pentanal mixture obtained in step a) is converted in the presence of basic compounds to a mixture of isomeric α,β-unsaturated decenals;
c) the mixture obtained in step b) is selectively hydrogenated in the presence of a hydrogenation catalyst with hydrogen at elevated temperature and elevated pressure to give a mixture of isomeric decanals; and
d) the mixture obtained in step c) is separated into a stream enriched with unconverted 2-methylbutanal and a stream enriched with a mixture of isomeric decanals; with the proviso that the stream which has been separated off in step d) and is enriched with unconverted 2-methylbutanal is reacted with formaldehyde.

8. Process according to Claim 7, **characterized in that** the reaction with formaldehyde is effected in the presence of a secondary amine and a mono-, di- or polycarboxylic acid.

9. Process according to Claim 8, **characterized in that** distillation is effected in the course of the reaction with formaldehyde.

10. Process according to one or more of Claims 7 to 9, **characterized in that** the 2-methylbutanal obtained in step d) is oxidized to 2-methylbutyric acid, hydrogenated to 2-methylbutanol or reductively aminated to 2-methylbutylamines.

11. Process according to Claim 1 or 7, **characterized in that** the reaction with formaldehyde is followed by distillative removal of 2-methylbutanal.

12. Process according to Claim 11, **characterized in that** the 2-methylbutanal obtained is oxidized to 2-methylbutyric acid, hydrogenated to 2-methylbutanal or reductively aminated to 2-methylbutylamines.

13. Process according to one or more of Claims 7 to 9, **characterized in that** the mixture of isomeric decanals obtained in step d) is hydrogenated to give a mixture of isomeric decanols, oxidized to give a mixture of isomeric decanoic acids or reductively aminated to give a mixture of isomeric decylamines.

## Revendications

1. Procédé de fabrication de 2-méthylbutanal à partir des courants secondaires formés lors de la fabrication de mélanges de décénals α,β-insaturés isomères, **caractérisé en ce que**
a) un mélange contenant des butènes linéaires est mis en réaction en présence de composés de métaux de transition du groupe VIII du tableau périodique des éléments avec du monoxyde de carbone et de l'hydrogène à température élevée et pression élevée pour former un mélange de pentanals ;
b) le mélange de pentanals obtenu selon l'étape a) est transformé en présence de composés basiques en un mélange de décénals α,β-insaturés isomères ; et
c) le mélange obtenu selon l'étape b) est séparé en un courant de matière enrichi en 2-méthylbutanal non réagi et en un courant de matière enrichi en un mélange de décénals α,β-insaturés isomères ; à condition que le courant de matière séparé selon l'étape c) et enrichi en 2-méthylbutanal non réagi soit mis en réaction avec du formaldéhyde.

2. Procédé selon la revendication 1, **caractérisé en ce que** la réaction avec du formaldéhyde a lieu en présence d'une amine secondaire et d'un acide mono-, di- ou polycarboxylique.

3. Procédé selon la revendication 2, **caractérisé en ce qu'**une distillation a lieu lors de la réaction avec du formaldéhyde.

4. Procédé selon une ou plusieurs des revendications 1 à 3, **caractérisé en ce que** le 2-méthylbutanal obtenu selon l'étape c) est oxydé en acide 2-méthylbutyrique, hydrogéné en 2-méthylbutanol ou aminé avec réduction en 2-méthylbutylamines.

5. Procédé selon une ou plusieurs des revendications 1 à 3, **caractérisé en ce que** le mélange de décénals α,β-insaturés isomères obtenu selon l'étape c) est hydrogéné complètement en un mélange de décanols isomères ou hydrogéné sélectivement en un mélange de décanals isomères.

6. Procédé selon la revendication 5, **caractérisé en ce que** le mélange de décanals isomères est oxydé en un mélange d'acides décanoïques isomères ou aminé avec réduction en un mélange de décylamines isomères.

7. Procédé de fabrication de 2-méthylbutanal à partir des courants secondaires formés lors de la fabrication de mélanges de décanals isomères, **caractérisé en ce que**
a) un mélange contenant des butènes linéaires est mis en réaction en présence de composés de métaux de transition du groupe VIII du tableau périodique des éléments avec du monoxyde de carbone et de l'hydrogène à température élevée et pression élevée pour former un mélange de pentanals ;
b) le mélange de pentanals obtenu selon l'étape a) est transformé en présence de composés basiques en un mélange de décénals α,β-insaturés isomères ;
c) le mélange obtenu selon l'étape b) est hydrogéné sélectivement en présence d'un catalyseur d'hydrogénation avec de l'hydrogène à température élevée et pression élevée en un mélange de décanals isomères ; et
d) le mélange obtenu selon l'étape c) est séparé en un courant de matière enrichi en 2-méthylbutanal non réagi et en un courant de matière enrichi en un mélange de décanals isomères ; à condition
que le courant de matière séparé selon l'étape d) et enrichi en 2-méthylbutanal non réagi soit mis en réaction avec du formaldéhyde.

8. Procédé selon la revendication 7, **caractérisé en ce que** la réaction avec du formaldéhyde a lieu en présence d'une amine secondaire et d'un acide mono-, di- ou polycarboxylique.

9. Procédé selon la revendication 8, **caractérisé en ce qu'**une distillation a lieu lors de la réaction avec du formaldéhyde.

10. Procédé selon une ou plusieurs des revendications 7 à 9, **caractérisé en ce que** le 2-méthylbutanal obtenu selon l'étape d) est oxydé en acide 2-méthylbutyrique, hydrogéné en 2-méthylbutanol ou aminé avec réduction en 2-méthylbutylamines.

11. Procédé selon la revendication 1 ou 7, **caractérisé en ce que** le 2-méthylbutanal est séparé par distillation après la réaction avec le formaldéhyde.

12. Procédé selon la revendication 11, **caractérisé en ce que** le 2-méthylbutanal obtenu est oxydé en acide 2-méthylbutyrique, hydrogéné en 2-méthylbutanal ou aminé avec réduction en 2-méthylbutylamines.

13. Procédé selon une ou plusieurs des revendications 7 à 9, **caractérisé en ce que** le mélange de décanals isomères obtenu selon l'étape d) est hydrogéné en un mélange de décanols isomères, oxydé en un mélange d'acides décanoïques isomères ou aminé avec réduction en un mélange de décylamines isomères.
